# EUROPEAN PATENT APPLICATION

(11) **EP 4 796 628 A1**
(43) Date of publication of application: **26.08.2026**
(21) Application number: 24881697.7
(22) Date of filing: 24.10.2024
(51) Int. Cl.: C12N 1/19, C12N 15/81, C12N 15/53, C07K 14/395, C12P 7/56

(54) **ACID-RESISTANT KLUYVEROMYCES MARXIANUS STRAIN AND USE THEREOF**

(30) Priority: 24.10.2023 CN 202311380695
(71) Applicant: China Petroleum & Chemical Corporation, Beijing 100728 (CN); Sinopec Dalian Research Institute of Petroleum and Petrochemicals Co., Ltd., Lushunkou District Dalian, Liaoning 116045 (CN)
(72) Inventor: ZHANG, Lin, Dalian, Liaoning 116045 (CN); ZHAO, Xinqing, Dalian, Liaoning 116045 (CN); FAN, Yachao, Dalian, Liaoning 116045 (CN); LIAO, Sha, Dalian, Liaoning 116045 (CN)
(74) Representative: karo IP
(86) International application number: PCT/CN2024/127063
(87) International publication number: WO 2025/087335

(57) **Abstract**

The present invention relates to the technical field of microorganisms, and in particular to an acid-resistant Kluyveromyces marxianus strain and the use thereof. The provided Kluyveromyces marxianus DLY-KMPMFF-0329 is deposited in the China General Microbiological Culture Collection Center with a deposit number of CGMCC No.27009. The strain can tolerate a high concentration of succinic acid and low pH conditions, can efficiently use glucose to synthesize succinic acid by means of the reductive tricarboxylic acid cycle pathway, and can achieve a relatively high production and yield of succinic acid. In addition, the strain has a relatively high homologous recombination efficiency, so that efficient gene editing can be achieved. The strain can be used not only as a succinic acid-producing strain for the fermentation production of succinic acid, but also as a starting strain for breeding or constructing a high-yield succinic acid-producing strain, and has a good application value.

## Description

### CROSS-REFERENCE TO RELATED APPLICATIONS

The application claims the benefit of the China patent application No. "202311380695.6", filed on October 24, 2023, the content of which is specifically and entirely incorporated herein by reference.

### TECHNICAL FIELD

The present invention relates to the technical field of microorganism, in particular to an acid-resistant *Kluyveromyces marxianus* strain and the use thereof.

### BACKGROUND ART

*Kluyveromyces cesmarxianus* has the characteristics of high temperature resistance, fast growth rate, natural utilization of multiple carbon sources such as xylose, lactose and inulin, thus is a potentially favorable host for production of bio-based chemicals (Qiu, et al., Kluyveromyces as promising yeast cell factors for industrial bioproduction: From bio-functional design to applications. Biotechnology Advances, 2023, 64:108-125).

Butanedioic acid, also known as succinic acid (SA), is one of the important bio-based chemical raw materials and has important applications in agriculture, food, clothing, cosmetics and pharmaceutical industries. In addition to being used as a surfactant, ion chelator and food additive, butanedioic acid can also be used as a precursor for many high-value industrial chemicals including adipic acid, 1,4-butanediol (1,4-BDO), tetrahydrofuran (THF), n-methylpyrrolidone (NMP), n-butyl lactone and precursor of other solvents and chemicals. Moreover, butanedioic acid can also be used for producing biodegradable materials such as polyesters and polyamides. In 2004 and 2010, the United States Department of Energy (DOE) listed butanedioic acid as one of the five most promising bio-based platform chemicals (Ahn, et al., Production of succinic acid by metabolically engineered microorganisms. Current Opinion in Biotechnology, 2016, 42, 54-66). In recent years, the demand for butanedioic acid as a platform chemical has grown rapidly. Therefore, it is of great significance to improve the production efficiency of succinic acid and reduce the production cost.

Compared with the production process based on raw materials source of fossil, the production of butanedioic acid by a microbial fermentation process is more competitive. At present, microorganisms that can produce butanedioic acid include *Yarrowia lipolytica*, *Saccharomyces cerevisiae*, *Escherichia coli*, succinic acid-producing *Actinobacillus succinogenes*, *Corynebacterium glutamicum*, etc., but there are relatively few studies on the fermentive production of butanedioic acid with *Kluyveromyces marxianus*, and *Kluyveromyces marxianus* is a yeast dominated by non-homologous recombination, which brings great difficulties to the efficient genetic manipulation of multiple gene sites therein.

Microorganisms can be used for producing SA through three pathways: the oxidative tricarboxylic acid cycle pathway (oTCA), the glyoxylic acid cycle pathway (GA), and the reductive tricarboxylic acid cycle pathway (rTCA). When production occurred simultaneously through the oTCA and GA pathways, pyruvic acid enters the mitochondria and generates acetyl-COA under the action of pyruvate dehydrogenase, and condenses with oxaloacetic acid to form citric acid, which is then oxidized and decarboxylated to form SA, with a theoretical yield of 1 mol SA / 1 mol glucose. In the rTCA pathway, after carboxylation of pyruvic acid or phosphoenolpyruvic acid, each molecule of the generated SA can fix 1 molecule of CO₂, so the theoretical yield is 1.71 mol SA / 1 mol glucose (Liu et al., Biosynthetic pathway and metabolic engineering of succinic acid. Frontiers in Bioengineering and Biotechnology, 2022, 10, 843887). Therefore, compared with oTCA, rTCA has a higher theoretical yield of SA production, and thus has greater application potential.

Currently, the main engineering host bacteria for producing SA through rTCA are bacteria such as *Escherichia coli*, *Actinomycetes*, *Corynebacterium glutamicum* and so on, however, these bacteria have a risk of being infected by bacteriophages, and bacteria have poor acid resistance (generally, the suitable pH range is 6.8-7.3, so that a large amount of neutralizer shall be added to neutralize the acidic environment caused by the production of butanedioic acid during the fermentation process, which increases the costs of fermentation and subsequent separation process. Yeast cells do not have the risk of being infected by bacteriophages and their acid resistance is generally better than that of bacteria. Therefore, using yeast cell as a chassis cell to produce SA through the rTCA pathway is a more promising production method. The reported SA production and yield of *Saccharomyces cerevisiae* via the rTCA pathway are both low. Although *Issatchenkia orientalis* naturally has a strong tolerance to low pH, by overexpression of endogenous pyruvate carboxylase PYC1, malate dehydrogenase MDH3, fumarate hydratase FUM1, and heterologous expression of the *Aspergillus oryzae*-derived fumarate reductase FRDg, the final SA production via the rTCA pathway was only 11.6 g/L, and the yield was only 0.12 g/g (Xiao et al., Exploiting Issatchenkiaorientalis SD108 for succinic acid production. Microbial Cell Factories, 2014, 13, 121).

At present, the production and yield of SA produced by yeast using glucose as a carbon source through oTCA or rTCA pathway are still low. One of the reasons is that the pH gradually decreases during the accumulation process of SA, which inhibits the acid production of the strain. According to literature reports, in experiments using yeast to produce malic acid, the production of malic acid was significantly improved by enhancing the tolerance of said production strain to low pH (Sun L et al., Highly efficient neutralizer-free L-malic acid production using engineered Saccharomyces cerevisiae. Bioresource Technology. 2023, 370: 128580), which demonstrates that the high tolerance of chassis cells to low pH is beneficial to the production of organic acids.

In summary, it is still necessary to develop production strains with higher acid resistance and butanedioic acid production capacity in order to further improve the yield and production efficiency of butanedioic acid.

### SUMMARY OF THE INVENTION

The present invention provides an acid-resistant *Kluyveromyces marxianus* strain and the use thereof.

During the research and development process, the applicant domesticated and screened *K. marxianus* NBRC1777 to improve its tolerance to low pH and butanedioic acid, and obtained a *K. marxianus* strain with strong tolerance to low pH and butanedioic acid; and used this strain as the parent strain, further enhanced the homologous recombination efficiency of the strain through genetic engineering, and obtained *K. marxianus* DLY-KMSP-0209. The strain was deposited in China General Microbiological Culture Collection Center (referred to as CGMCC, address: No. 3, Courtyard 1 Beichen West Road, Chaoyang District, Beijing, Institute of Microbiology, Chinese Academy of Sciences, Postal Code 100101) on February 16, 2022, and the classification name is *Kluyveromyces marxianus*, with a deposit number of CGMCC No. 24398. *K. marxianus* DLY-KMSP-0209 can tolerate low pH conditions and high concentration of butanedioic acid. Moreover, the strain has high homologous recombination efficiency and is suitable as a chassis cell for breeding fermentive production strains of butanedioic acid and other organic acids. In addition, *K. marxianus* DLY-KMSP-0209 can be grown using one or more selected from glucose, lignocellulosic biomass (including xylose, lignocellulose hydrolysate, etc.) as a carbon source, and the suitable growth temperature is 30-42°C (preferably 37-42°C), which can achieve tolerance to low pH and high succinic acid concentrations at a high temperature for fermentive production.

To further improve the yield of succinic acid, the present invention uses *Kluyveromyces marxianus* DLY-KMSP-0209 as a parent strain, firstly performs domestication of low pH and butanedioic acid tolerance on *Kluyveromyces marxianus* DLY-KMSP-0209, further performs genetic engineering modification on the dominant strain obtained by domestication and screening so that the strain can produce butanedioic acid through reductive TCA pathway (rTCA), and finally implements glucose substrate-oriented screening on the genetically engineered strain to obtain a strain that can efficiently utilize glucose to synthesize butanedioic acid, and finally obtains a *Kluyveromyces marxianus* strain with good tolerance of low pH and succinic acid, high capability to synthesize butanedioic acid by using glucose, and the ability to produce butanedioic acid by using rTCA, and with the high production and yield of butanedioic acid, which is named DLY-KMPMFF-0329.

Specifically, the present invention provides the following technical solutions:
In the first aspect, the present invention provides *Kluyveromyces marxianus* DLY-KMPMFF-0329, which was deposited in China General Microbiological Culture Collection Center (referred to as CGMCC, address: No. 3, Courtyard 1 Beichen West Road, Chaoyang District, Beijing, Institute of Microbiology, Chinese Academy of Sciences, Postal Code 100101) on April 3, 2023, and the classification name is *Kluyveromyces marxianus*, with a deposit number of CGMCC No. 27009.

The invention constructs rTCA in *Kluyveromyces marxianus* DLY-KMPMFF-0329 through genetic engineering modification, and the specific modifications comprise: making fumaratec reductase FRDg with an amino acid sequence as set forth in SEQ ID NO.4 be expressed, enhancing the expression of pyruvate carboxylase PYC2, making malate dehydrogenase MDH3 with an amino acid sequence as set forth in SEQ ID NO.2 be expressed, making fumarate hydratase FUM1 with an amino acid sequence as set forth in SEQ ID NO.3 be expressed, inactivating the encoding gene of malic enzyme MAE1, and inactivating the encoding gene of pyruvate decarboxylase PDC1.

Therefore, the *Kluyveromyces marxianus* DLY-KMPMFF-0329 has the following genotype: expressing fumaratec reductase FRDg with an amino acid sequence as set forth in SEQ ID NO.4 and/or, its genome contains a DNA fragment with a nucleotide sequence as set forth in SEQ ID NO.5.

In addition, compared with the wild type *Kluyveromyces marxianus* NBRC1777, the *Kluyveromyces marxianus* DLY-KMPMFF-0329 has the enhanced expression of pyruvate carboxylase PYC2, and/or, expresses the malate dehydrogenase MDH3 with an amino acid sequence as set forth in SEQ ID NO.2, and/or, expresses the fumarate hydratase FUM1 with an amino acid sequence as set forth in SEQ ID NO.3, and/or, inactivates the encoding gene of malic enzyme MAE1, and/or, inactivates the encoding gene of pyruvate decarboxylase PDC1.

The *Kluyveromyces marxianus* DLY-KMPMFF-0329 can tolerate low pH conditions and high butanedioic acid concentrations, allowing the strain to adapt to more harsh production conditions and avoiding product suppression phenomenon, thereby obtaining the higher production and yield when using the strain for fermentive production of butanedioic acid. Moreover, the strain has high homologous recombination efficiency and is suitable as a chassis cell for breeding fermentive production strains of organic acids such as butanedioic acid. In addition, *Kluyveromyces marxianus* DLY-KMPMFF-0329 can efficiently utilize glucose to synthesize succinic acid through rTCA, and has a higher production and yield of butanedioic acid.

In the second aspect, the present invention provides a *Kluyveromyces marxianus* comprising a gene expressing a fumaratec reductase FRDg derived from *Trypanosoma brucei*.

According to a preferred embodiment of the present invention, wherein amino acid sequence of the fumaratec reductase FRDg has identity not less than 70%, preferably not less than 80%, more preferably not less than 90% with the amino acid sequence as set forth in SEQ ID No. 4. Preferably, activity of the fumarate reductase FRDg is more than 70%, more preferably more than 80%, even more preferably more than 90% of activity of the fumaratec reductase FRDg with the amino acid sequence as set forth in SEQ ID No. 4. Preferably, the fumaraic reductase FRDg comprises 1,000-1,300 amino acids.

Preferably, an amino acid sequence of the fumaratec reductase FRDg has identity of 95-100% with the amino acid sequence as set forth in SEQ ID No. 4. For example, the identity may be 95%, 96%, 97%, 98%, 99%, 99.1%, 99.2%, 99.3%, 99.4%, 99.5%, 99.6%, 99.7%, 99.8%, 99.9%, 100%, or it may fall into a range consisting of any two of the above values, or may be a random intermediate value in the range.

According to a preferred embodiment of the invention, wherein genome of the *Kluyveromyces marxianus* includes an encoding gene of fumaratec reductase FRDg with a nucleotide sequence having identity more than 60% with the nucleotide sequence as set forth in SEQ ID No. 5. Preferably, encoding gene of the fumaratec reductase FRDg has identity not less than 70%, more preferably not less than 80%, further preferably not less than 90% with the nucleotide sequence as set forth in SEQ ID No. 5.

Preferably, encoding gene of the fumaraic reductase FRDg has identity of 95-100% with the nucleotide sequence as set forth in SEQ ID No. 5. For example, the identity may be 95%, 96%, 97%, 98%, 99%, 99.1%, 99.2%, 99.3%, 99.4%, 99.5%, 99.6%, 99.7%, 99.8%, 99.9%, 100%, or it may fall into a range consisting of any two of the above values, or may be a random intermediate value in the range.

According to a preferred embodiment of the invention, compared with a wild type *Kluyveromyces marxianus*, the *Kluyveromyces marxianus* has the enhanced expression of pyruvate carboxylase PYC2, and/or, expresses the malate dehydrogenase MDH3, and/or, expresses the fumarate hydratase FUM1, and/or, inactivates the encoding gene of malic enzyme MAE1, and/or, inactivates the encoding gene of pyruvate decarboxylase PDC1.

Preferably, amino acid sequence of the malate dehydrogenase MDH3 has identity not less than 70%, preferably not less than 80%, more preferably not less than 90% with the amino acid sequence as set forth in SEQ ID No. 2.

More preferably, amino acid sequence of the malate dehydrogenase MDH3 has identity of 95-100% with the amino acid sequence as set forth in SEQ ID NO: 2. For example, the identity may be 95%, 96%, 97%, 98%, 99%, 99.1%, 99.2%, 99.3%, 99.4%, 99.5%, 99.6%, 99.7%, 99.8%, 99.9%, 100%, or it may fall into a range consisting of any two of the above values, or may be a random intermediate value in the range.

Preferably, amino acid sequence of the fumarate hydratase FUM1 has identity not less than 70%, more preferably not less than 80%, further preferably not less than 90% with the amino acid sequence as set forth in SEQ ID No. 3.

More preferably, the fumarate hydratase FUM1 has an amino acid sequence with 95-100% identity with the amino acid sequence as set forth in SEQ ID No. 3. For example, the identity may be 95%, 96%, 97%, 98%, 99%, 99.1%, 99.2%, 99.3%, 99.4%, 99.5%, 99.6%, 99.7%, 99.8%, 99.9%, 100%, or it may fall into a range consisting of any two of the above values, or may be a random intermediate value in the range.

According to one preferred embodiment of the invention, wherein the wild type *Kluyveromyces marxianus* is NBRC1777.

According to some preferred embodiments of the invention, wherein the *Kluyveromyces marxianus* differs from *Kluyveromyces marxianus* with a deposit number of CGMCC No.24398 in the inactivated encoding gene of malic enzyme MAE1.

According to some preferred embodiments of the invention, wherein the *Kluyveromyces marxianus* differs from *Kluyveromyces marxianus* with a deposit number of CGMCC No.24398 in the inactivated encoding gene of pyruvate decarboxylase PDC1.

Preferably, the *Kluyveromyces marxianus* is DLY-KMPMFF-0329 with a deposit number of CGMCC No 27009.

Further, the present invention provides a method for preparing a *Kluyveromyces marxianus* engineered strain with high butanedioic acid yield, wherein the method comprises introducing a gene expressing a fumaratec reductase FRDg derived from *Trypanosoma brucei* into the genome of a wild type *Kluyveromyces marxianus*.

Preferably, amino acid sequence of the fumaratec reductase FRDg has identity not less than 70%, preferably not less than 80%, more preferably not less than 90% with the amino acid sequence as set forth in SEQ ID No. 4.

Preferably, amino acid sequence of the fumaratec reductase FRDg has identity of 95-100% with the amino acid sequence as set forth in SEQ ID No. 4. For example, the identity may be 95%, 96%, 97%, 98%, 99%, 99.1%, 99.2%, 99.3%, 99.4%, 99.5%, 99.6%, 99.7%, 99.8%, 99.9%, 100%, or it may fall into a range consisting of any two of the above values, or may be a random intermediate value in the range.

According to preferred embodiments of the invention, wherein encoding gene of the fumaratec reductase FRDg has identity more than 60% with the nucleotide sequence as set forth in SEQ ID No. 5. Preferably, the encoding gene of the fumaratec reductase FRDg has identity not less than 70%, more preferably not less than 80%, further preferably not less than 90% with the nucleotide sequence as set forth in SEQ ID No. 5.

Preferably, encoding gene of the fumaratec reductase FRDg has identity of 95-100% with the nucleotide sequence as set forth in SEQ ID No. 5. For example, the identity may be 95%, 96%, 97%, 98%, 99%, 99.1%, 99.2%, 99.3%, 99.4%, 99.5%, 99.6%, 99.7%, 99.8%, 99.9%, 100%, or it may fall into a range consisting of any two of the above values, or may be a random intermediate value in the range.

According to preferred embodiments of the invention, wherein the method further comprises: compared with the wild type *Kluyveromyces marxianus*, causing that the *Kluyveromyces marxianus* engineered strain has an enhanced expression of pyruvate carboxylase PYC2, and/or, causing that the *Kluyveromyces marxianus* engineered strain expresses the malate dehydrogenase MDH3, and/or, causing that the *Kluyveromyces marxianus* engineered strain expresses the fumarate hydratase FUM1, and/or, inactivating the encoding gene of malic enzyme MAE1 in the *Kluyveromyces marxianus* engineered strain, and/or, inactivating the encoding gene of pyruvate decarboxylase PDC1 in the *Kluyveromyces marxianus* engineered strain.

Preferably, amino acid sequence of the malate dehydrogenase MDH3 has identity not less than 70%, preferably not less than 80%, more preferably not less than 90% with the amino acid sequence as set forth in SEQ ID No. 2.

More preferably, amino acid sequence of the malate dehydrogenase MDH3 has identity of 95-100% with the amino acid sequence as set forth in SEQ ID No. 2. For example, the identity may be 95%, 96%, 97%, 98%, 99%, 99.1%, 99.2%, 99.3%, 99.4%, 99.5%, 99.6%, 99.7%, 99.8%, 99.9%, 100%, or it may fall into a range consisting of any two of the above values, or may be a random intermediate value in the range.

Preferably, amino acid sequence of the fumaratic hydratase FUM1 has identity not less than 70%, preferably not less than 80%, more preferably not less than 90% with the amino acid sequence as set forth in SEQ ID No. 3.

More preferably, amino acid sequence of the fumarate hydratase FUM1 has identity of 95-100% with the amino acid sequence as set forth in SEQ ID No. 3. For example, the identity may be 95%, 96%, 97%, 98%, 99%, 99.1%, 99.2%, 99.3%, 99.4%, 99.5%, 99.6%, 99.7%, 99.8%, 99.9%, 100%, or it may fall into a range consisting of any two of the above values, or may be a random intermediate value in the range.

In the present invention, a gene may be inactivated with a conventional manner common in the field, and inactivation of a gene refers to that the gene is expressed in an amount not exceeding 5% of the original expression amount, preferably the gene is not expressed completely. The particular manner of inactivation may be a (full) gene knock-out, a replacement/knock-out of expression control region of the gene, or an introduction of other gene sequences that can inhibit the expression of the gene, etc.

According to a particularly preferred embodiment of the invention, the method comprises:
(1) Subjecting the wild type *Kluyveromyces marxianus* to domestication, screening and genetic modification to obtain a chassis strain with improved homologous recombination efficiency;
(2) Subjecting the chassis bacterium obtained in step (1) to domestication, screening and genetic modification to obtain the *Kluyveromyces marxianus* engineered strain.

Preferably, homologous recombination efficiency of the chassis strain in step (1) is increased by at least 0.5%, more preferably increased by 0.5-90%, compared with the wild type *Kluyveromyces marxianus*. For example, the homologous recombination efficiency of the chassis strain may be increased by 0.5%, 1%, 2%, 3%, 4%, 5%, 10%, 15%, 20%, 25%, 30%, 35%, 40%, 45%, 50%, 55%, 60%, 65%, 70%, 75%, 80%, 85%, 90%, compared with the wild type *Kluyveromyces marxianus*, or it may fall into a range consisting of any two of the above values, or may be a random intermediate value in the range.

Preferably, the wild type *Kluyveromyces marxianus* is NBRC1777.

More preferably, the chassis bacterium is the *Kluyveromyces marxianus* DLY-KMSP-0209 with a deposit number of CGMCC No. 24398.

The invention further provides a *Kluyveromyces marxianus* engineered strain obtained according to the aforementioned method.

In the third aspect, the invention also provides a microorganism preparation comprising the aforementioned *Kluyveromyces marxianus*.

According to a preferred embodiment of the invention, wherein the *Kluyveromyces marxianus* is DLY-KMPMFF-0329.

The aforementioned microorganism preparations may be a solid preparation or a liquid preparation. *Kluyveromyces marxianus* in the microorganism preparation is present in the form of a viable organism. The microorganism preparation may comprise a carrier or other auxiliary ingredients which are allowed in the field of microorganism preparation, in addition to *Kluyveromyces marxianus* or a culture of *Kluyveromyces marxianus*.

Preferably, the auxiliary ingredients comprise at least one selected from the group consisting of a protective agent (e.g., a lyoprotectant), a buffering agent and a stabilizer.

The invention also provides a method for preparing the aforementioned microorganism preparation, wherein the method comprises a step of culturing the *Kluyveromyces marxianus*.

In some embodiments of the invention, the *Kluyveromyces marxianus* is cultured to produce a culture solution, which is directly prepared into a liquid microorganism preparation or mixed with an auxiliary ingredient to prepare into a liquid microorganism preparation, or the culture solution is mixed with an auxiliary ingredient directly and lyophilized to produce a solid microorganism preparation, or the culture solution is subjected to a solid-liquid separation to obtain a thallus, which is mixed with an auxiliary ingredient and lyophilized to produce a solid microorganism preparation.

In the fourth aspect, the invention provides a use of the aforementioned *Kluyveromyces marxianus* or the microorganism preparations in anyone as following:
(1) use in fermentive production of an organic acid or derivatives thereof;
(2) use in breeding and/or constructing production strain of an organic acid or derivatives thereof.

In use (2) mentioned above, breeding or constructing a production strain of organic acid or derivatives thereof may be carried out by using *Kluyveromyces marxianus* provided by the present invention (e.g. *Kluyveromyces marxianus* as described in the first aspect or the second aspect above, or *Kluyveromyces marxianus* constructed according to the above-described method) as a parent strain. Among these, breeding refers to obtaining a new strain by using the non-rational modification methods such as mutagenesis or domestication; and constructing refers to obtaining a new strain by using the rational modification methods such as genetic engineering. Derivatives of organic acid may be derivative compounds such as salts (e.g. ammonium salts, metal salts), esters, which are produced by using organic acid as a main substrate.

In the invention, the parent strain refers to an original strain used for breeding or constructing the yeast species, and the parent strain is subjected to genetic engineering, mutagenesis or domestication to obtain the desired strain.

The *Kluyveromyces marxianus* provided according to the first and second aspects of the invention, especially *Kluyveromyces marxianus* DLY-KMPMFF-0329, has a high tolerance to low pH and butanedioic acid, at the same time, it has a high homologous recombination efficiency, and also efficiently utilizes glucose for producing butanedioic acid via the rTCA pathway having a higher theoretical yield. Therefore, the strain is suitable as a parent strain for breeding or constructing the production strains for an organic acid (especially butanedioic acid) or derivatives thereof.

Preferably, the organic acid is butanedioic acid.

The invention further provides a method for constructing a *Kluyveromyces marxianus* engineered strain for producing butanedioic acid, wherein the method comprises: using the *Kluyveromyces marxianus* provided by the invention (e.g., *Kluyveromyces marxianus* DLY-KMPMFF-0329) as a parent strain, and subjecting the parent strain to a genetic modification.

Preferably, the genetic modification comprises: introducing an exogenous nucleic acid into the parent strain by using the homologous recombination technologies, and/or subjecting the endogenous gene of the parent strain to a gene editing by using the homologous recombination technologies.

The homologous recombination technologies include, but are not limited to, CRISPR/Cas9, Cre-Loxp, TALENs and the like.

The fifth aspect of the invention provides a method for fermentive production of butanedioic acid, wherein the method comprises a step of culturing the *Kluyveromyces marxianus* according to the first aspect or the second aspect, or the *Kluyveromyces marxianus* prepared by the method according to the third aspect.

Preferably, temperature of the culturing is within the range of 37-42°C.

According to a preferred embodiment of the invention, wherein the *Kluyveromyces marxianus* is *Kluyveromyces marxianus* DLY-KMPMFF-0329 with a deposit number of CGMCC No. 27009.

Preferably, the culturing comprises a fermentation stage comprising a step of culturing the *Kluyveromyces marxianus* DLY-KMPMFF-0329 under oxygen-limited conditions.

The oxygen-limited conditions serve to control dissolved oxygen DO to below 2%. Preferably, the dissolved oxygen DO is controlled to below 1%. For example, the dissolved oxygen DO may be controlled at 2%, 1.8%, 1.5%, 1.2%, 1%, 0.9%, 0.8%, 0.7%, 0.6%, 0.5%, 0.4%, 0.3%, 0.2%, 0.1%, or it may be controlled within a range consisting of any two of the above values, or may be controlled to be a random intermediate value within the range.

The invention has discovered that during the production process of butanedioic acid by using the rTCA pathway, the dissolved oxygen level will impose an obvious influence on the fermentation process, the conversion of butanedioic acid, and the formation of by-products. Controlling the dissolved oxygen level under the oxygen-limited conditions described above is conducive to the synthesis of butanedioic acid and reduced generation of by-products.

Preferably, during the fermentation cultivation stage, carbonate is added at the fermentation initiation stage and/or during fermentation process; the carbonate is added in an amount of 10-50g/L.

The present invention has found that during the production process of butanedioic acid by using the rTCA pathway, the immobilisation of carbon dioxide can be achieved by means of adding carbonate. Carbon dioxide can be used by *Kluyveromyces marxianus* DLY-KMPMFF-0329 in a form of carbonate (e.g. calcium carbonate), which can significantly improve the production of butanedioic acid, and provides a new idea and solution for the issue of carbon emission reduction.

The carbonate described above preferably comprises at least one selected from the group consisting of calcium carbonate, sodium carbonate, potassium carbonate and ammonium carbonate. Most preferably, the carbonate is calcium carbonate.

Preferably, carbonate may be added directly to the fermentation medium, and/or, carbonate may be added all at once or in multiple batches during the fermentation process. The carbonate is added in the total amount of 10-50g/L when carbonate is added in multiple batches.

The medium used in the fermentation culture described above includes a carbon source and a nitrogen source; the carbon source includes glucose, and the nitrogen source includes peptone and/or yeast powder.

Preferably, mass ratio of the carbon source to the nitrogen source is (3-6):1 (calculated in terms of C and N, the ratio may also be referred to as "carbon-nitrogen ratio"). The present invention has discovered that an appropriate increase of carbon-nitrogen ratio in the fermentation medium is advantageous for increasing the production of butanedioic acid through the fermentive production of *Kluyveromyces marxianus* DLY-KMPMFF-0329, the carbon-nitrogen ratio is preferably controlled within the range of (3-6): 1.

Preferably, the fermentation medium comprises: glucose 20-100g/L, peptone 0-20g/L, and yeast powder 5-15g/L.

Preferably, the fermentation cultivation stage comprises a first fermentation cultivation stage and a second fermentation cultivation stage; wherein the first fermentation cultivation stage relates to culturing under aerobic conditions, and the second fermentation cultivation stage relates to culturing under oxygen-limited conditions.

The present inventors have found that fermentation of *Kluyveromyces marxianus* DLY-KMPMFF-0329 by using a two-stage fermentation process (the first stage is mainly used for thallus growth and the second stage is mainly used for production of succinic acid) is more advantageous for promoting the production of butanedioic acid, further significantly increasing the fermentation level and shortening the fermentation period.

The culturing may further comprise a seed cultivation stage.

The invention at least includes the following favorable effects: the *Kluyveromyces marxianus* DLY-KMPMFF-0329 provided by the invention can tolerate high concentration of butanedioic acid and low pH conditions, can efficiently utilize glucose for the synthesis of butanedioic acid via the rTCA pathway, can achieve improved production and yield of butanedioic acid, and has higher homologous recombination efficiency, and can efficiently perform the gene editing. On this basis, the *Kluyveromyces marxianus* DLY-KMPMFF-0329 can be either used as a butanedioic acid-producing strain for carrying out the fermentive production of butanedioic acid, or can be used as a parent strain for breeding or constructing succinic acid hyper-producing strain, and has better application value.

### BRIEF DESCRIPTION OF THE DRAWINGS

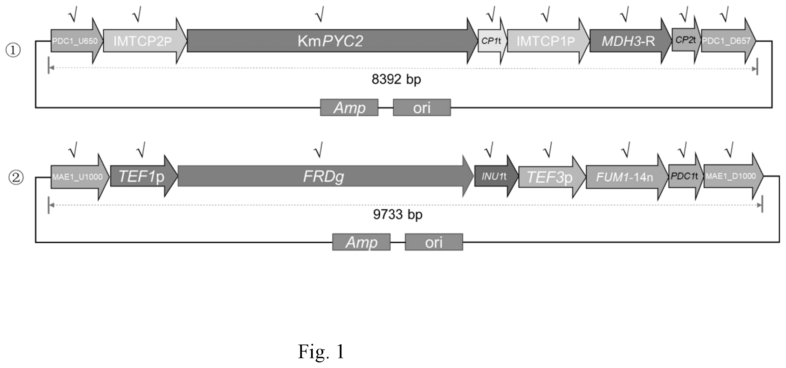

FIG. 1 is a schematic diagram of the expression cassettes used for reductive TCA pathway construction in Example 2 of the present invention, wherein IMTCP2p-km*PYC*2-CP1t indicates overexpression of *Kluyveromyces marxianus* endogenous pyruvate carboxylase gene kmPYC2 by using the *Kluyveromyces marxianus* endogenous promoter IMTCP2p and terminator IMTCP1 (indicated by CP1t in the diagram); IMTCP1p-*MDH*3-R-CP2t indicates overexpression of *Kluyveromyces marxianus* endogenous malate dehydrogenase gene MDH3-R (3 amino acids "TKL" at the C-terminal are deleted) by using the *Kluyveromyces marxianus* endogenous promoter IMTCP1p and terminator IMTCP2 (indicated by CP2t); the gene segments PDC1_U650 and PDC1_D657 indicate homology arm (used for targeting the desired segment *PDC1*) 650bp upstream and 657bp downstream the coding cassette of the gene PDC1; the segment *TEF1*p-*FRD*g-*INU*t indicates heterologous overexpression of fumaratec reductase FRDg with the *Kluyveromyces marxianus* endogenous promoter *TEF1*p and *INU1* gene terminator *INU1*t, *TEF3*p-*FUM*1-14n-*PDC1*t indicates an expression of endogenous fumarate hydratase (14n ind*ica*t*es* mitochondrial localization sequence with 14 amino acids deleted) with the *Kluyveromyces marxianus* endogenous promoter *TEF3*p and *PDC1* gene terminator *PDC1*t, the gene segments MAE1_U1000 and MAE1_D1000 indicate homology arms 1,000 bp upstream and downstream the malate dehydrogenase gene (MAE1) for targeting a large fragment at the location of gene MAE 1.

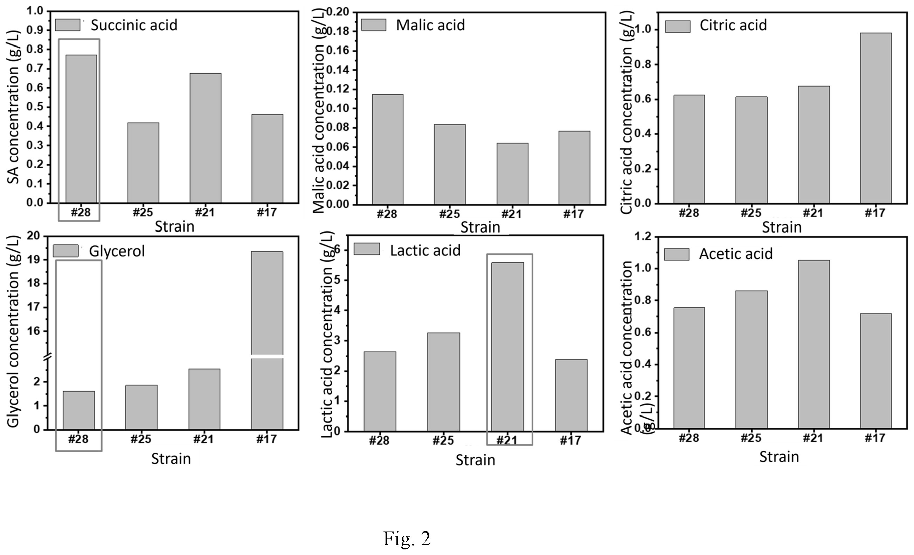

FIG. 2 shows the result of metabolite assay for glucose substrate directed screening experiments conducted on the 4 transformants having the correct genotype in Example 3 of the invention, wherein #28, #25, #21, #17 represent the 4 transformants having the correct genotype.

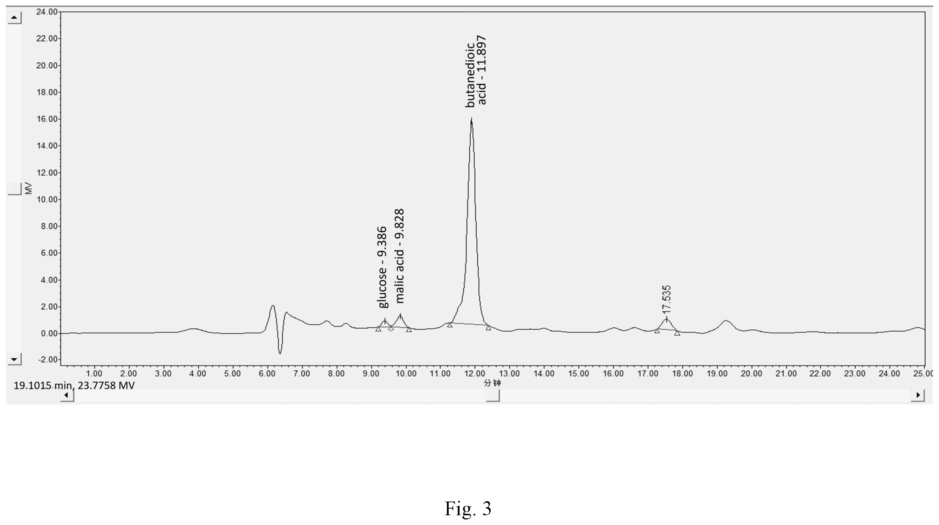

FIG. 3 shows the detection result of butanedioic acid production and accumulation of by-products by microaerobic fermentation of the *Kluyveromyces marxianus* DLY-KMPMFF-0329 in Example 4 of the invention, wherein the peak appearance time for butanedioic acid is 11.903 min.

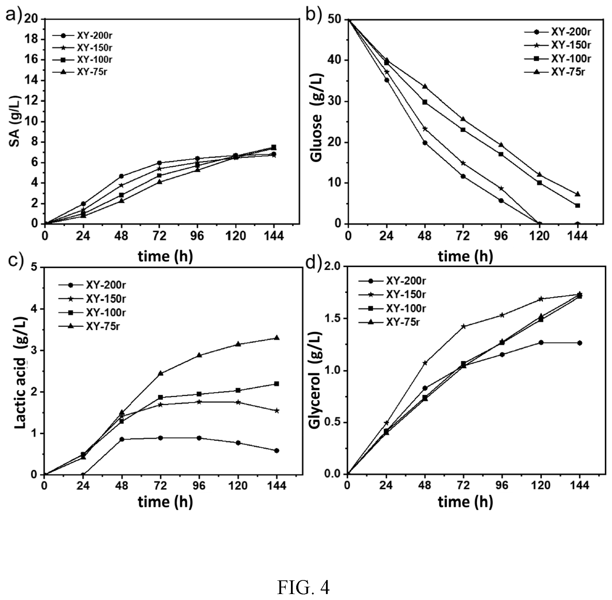

FIG. 4 shows the effect of different rotational speeds on butanedioic acid production under oxygen-limited conditions in Example 5, wherein a) illustrates the butanedioic acid production, b) illustrates the glucose consumption, c) illustrates the lactic acid accumulation, and d) shows the glycerol accumulation.

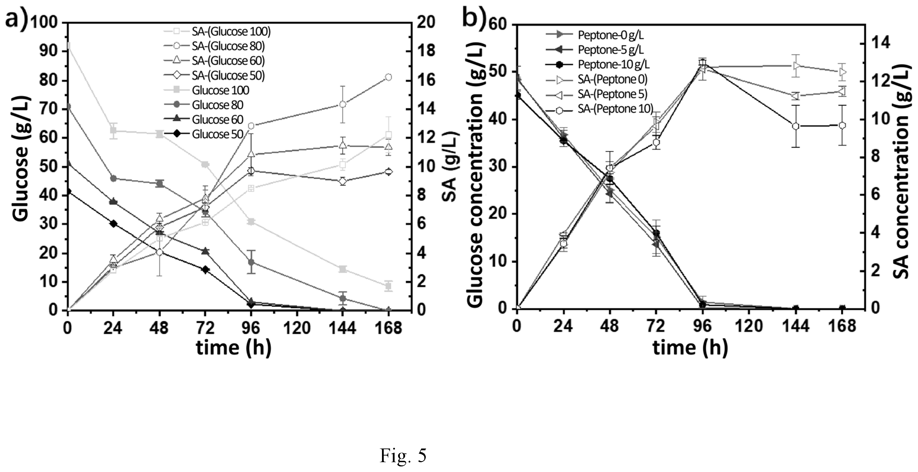

FIG. 5 shows the effect of shake flask culture condition optimization on the butanedioic acid production in Example 6 of the invention, wherein a) illustrates an influence of different dextrose concentrations on butanedioic acid accumulation, b) shows an influence of different peptone concentrations on butanedioic acid accumulation, wherein the solid marker curve represents the glucose consumption condition, the hollow marker curve represents the succinic acid production; glucose 50, 60, 80 and 100 represents the addition of glucose with the concentrations of 50 g/L, 60 g/L, 80 g/L and 100 g/L respectively; peptone-0 g/L, peptone-5 g/L, peptone-10 g/L denote that the peptone is not added, the addition of peptone with the concentrations of 5 g/L and 10g/L respectively.

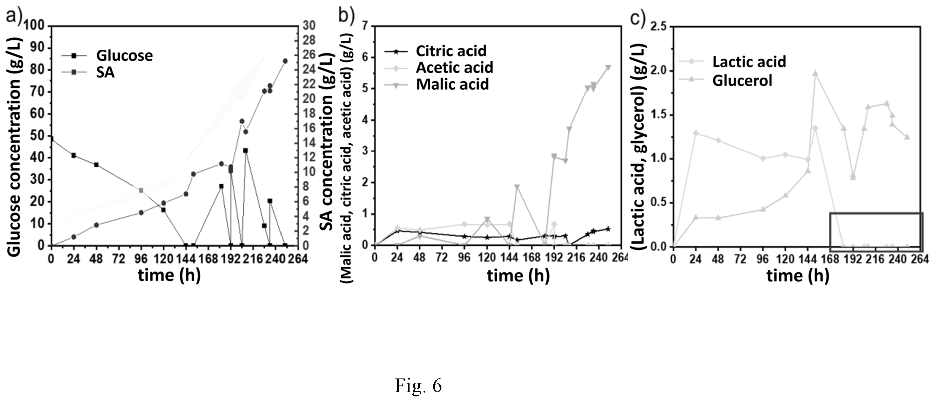

FIG. 6 shows an analysis of metabolites of butanedioic acid produced by 5L fermentor in Example 7 of the present invention, wherein a) illustrates the change of glucose versus SA; b) illustrates the accumulation of by-products citric acid, acetic acid and malic acid; and c) illustrates the accumulation of by-products glycerol and lactic acid. In FIGS. 1-6, SA represents butanedioic acid.

### DESCRIPTION OF THE PREFERRED EMBODIMENT

In order to clearly formulate the purpose, technical solution and advantages of the present invention, the technical solution of the invention will be explicitly and comprehensively described below with reference to the appended figures of the present invention. Obviously, the embodiments described herein are a part of the embodiments of the present invention, instead of all the embodiments. Based on the embodiments of the present invention, all other embodiments obtained by the ordinary technicians in the field without paying a creative labor fall into the protection scope of the present invention.

### Example 1 Low pH and butanedioic acid tolerance acclimation of Kluyveromyces marxianus

*Kluyveromyces marxianus* DLY-KMSP-0209 was used as the parent strain, and it was subjected to the low pH and succinic acid tolerance acclimation, the specific method was as follows: the strain was inoculated in 2 mL of YPD liquid medium and cultured under the conditions of 37°C and a rotational speed of 200 rpm until the culture broth began to become turbid, the strain was then inoculated in 1 mL of YPD liquid culture medium, and 3 mL of SA mother solution with a concentration of 60 g/L was added (the final concentration of SA was 45 g/L); the subculture was continued under the same conditions for 90 days; finally, the strain was inoculated in 0.5 mL of YPD liquid culture medium with a pH of 2.3 and added with 3.5 mL of SA mother solution with a concentration of 60 g/L (the final concentration of SA was 52.5 g/L) and the subculture was continued under the same conditions for 20 days, the bacterial solution was taken to spread on a flat plate, and a single clone with tolerance to low pH and high concentration butanedioic acid was selected to obtain the strain DLY-KMSP-0209M. The strain was capable of growing under the conditions of pH 2.3 and SA concentration of 52.5g/L. The tolerance test results showed that the OD₆₀₀ value of the strain DLY-KMSP-0209M in the logarithmic phase was 20% or higher than that of the parent strain DLY-KMSP-0209 under the same stress conditions.

### Example 2 Construction of a strain for producing butanedioic acid via the reductive TCA pathway

The production of butanedioic acid by using the reductive TCA pathway required four key genes, namely the gene that encoded the pyruvate carboxylase PYC2, which catalyzed pyruvic acid and HCO₃⁻ to produce oxaloacetic acid; the gene that encoded the malate dehydrogenase MDH3, which reduced oxaloacetic acid to malic acid under the condition of consuming one NADH molecule; the gene that encoded the fumarate hydratase FUM1, which converted malic acid into fumaric acid; and the gene that encoded fumaratec reductase FRDg, which reduced fumaric acid to butanedioic acid under the condition of consuming one NADH molecule. Among them, the first three genes of PYC2, MDH3 and FUM1 can be endogenously amplified from the *Kluyveromyces marxianus* NBRC1777 strain, but one of the key genes FRDg was derived from *Trypanosoma brucei*, which needed to be heterologously synthesized and codon optimized. The coenzyme of this enzyme was NADH, which was conducive to unifying the coenzyme to establish the redox balance.

Since the mitochondria of yeast cells had a double-layer membrane structure, the products (malic acid, fumaric acid, succinic acid) in the reductive TCA pathway required to make use of transporters to cross the double-layer membrane into the cytoplasm and then secreted to the outside of the cell, which undoubtedly hindered the production and exocytosis of succinic acid. However, the principle of transporting the above-mentioned organic dicarboxylic acids from mitochondria to the cytoplasm was not completely clear at present, in order to eliminate the effect as much as possible, the reduction pathway-related enzymes positioned in the mitochondria were directly transferred to the cytoplasm. PYC2p (amino acid sequence as shown in SEQ ID NO.1, encoded by the gene KLMA_10253) per se was a cytoplasmic protein, which was connected with the endogenous promoter IMTCP2p and terminator IMTCP1 of *Kluyveromyces marxianus*, and the IMTCP2p-km*PYC*2-CP1t expression cassette was constructed, and inserted into the genomic position where the *PDC1* gene was located. MDH3p was localized in peroxisomes, and the three amino acids "TKL" at the C-terminus of MDH3p (the amino acid sequence was shown in SEQ ID NO.2) were removed and placed in the IMTCP1p-*MDH*3-R-CP2t expression cassette for cytoplasmic expression. The protein FUM1p (encoded by gene KLMA_80028) dually-localized in mitochondria and cytoplasm was removed 14 amino acids (mitochondrial localization sequence included 14 amino acids and 1 start amino acid, the amino acid sequences were as shown in SEQ ID NO.3) from the N-terminus, and a new translation start codon was introduced to localize it in the cytoplasm, and expressed with the *TEF3*p-*FUM*1-14n-*PDC1*t expression cassette. The three "SKI" amino acids at the C-terminus of FRDgp were removed (the amino acid sequence was as set forth in SEQ ID NO.4, and the coding gene sequence was as set forth in SEQ ID NO.5), and connected with the endogenous promoter TEF1p and the INU1 gene terminator INU1t of *Kluyveromyces marxianus* to obtain the TEF1p-FRDg-INUt expression cassette so that it can perform the cytoplasmic expression.

In addition, the malic enzyme encoded by the gene MAE1 (NCBI-GeneID: 34717552) can decarboxylate the oxaloacetic acid to pyruvic acid, which resulted in an ineffective cycle, so MAE1 was further inactivated. Considering that *Kluyveromyces marxianus* may produce a large amount of ethanol during the cultivation process at the high glucose concentration, the gene PDC1 (NCBI-GeneID: 34717303) encoding pyruvate decarboxylase was further inactivated.

In summary, the advantageous strain DLY-KMSP-0209M with tolerance to low pH and butanedioic acid obtained by domestication in Example 1 was subjected to the following genetic manipulations to construct a cytoplasmic reductive TCA pathway: four key genes of the reductive TCA pathway (namely the gene encoding pyruvate carboxylase PYC2p, the gene encoding malate dehydrogenase MDH3-R, the gene encoding fumarate hydratase FUM1, and fumarate reductase FRDg, having the amino acid sequences as set forth in SEQ ID NO. 1-4 respectively) were overexpressed, and genes MAE1 and PDC1 were destroyed. An expression cassette plasmid was constructed for the established cytoplasmic reductive TCA pathway for producing succinic acid, and a schematic diagram of the plasmid was shown in FIG. 1. The above-mentioned genetic manipulations were completed by using the CRISPR/cas9 genome editing technology, 4 transformants with correct genotypes were obtained, which were named as #17, #21, #25, and #28 transformants, respectively.

### Example 3 Directed screening of glucose substrates in a strain for producing butanedioic acid by using the reductive TCA pathway

Initially, the transformants #17, #21, #25, and #28 obtained from the above screening process hardly grew under anaerobic conditions due to the destruction of *PDC1*.

However, after two generations of transfer under aerobic conditions, they resumed growth under aerobic conditions, but the lag phase and growth cycle were slightly extended.

First, the transformants #17, #21, #25, and #28 were placed in an aerobic YPD culture medium (composed of 10 g/L of yeast powder, 20 g/L of peptone, and 20 g/L of glucose) under the conditions of 37°C and a rotational speed of 200 rpm to quickly obtain a large number of thallus, which were then collected by centrifugation, and transferred to a fresh YPD medium, and 10 g/L CaCO₃ was added for anaerobic fermentation (placed in 80 mL round-bottom centrifuge tube, sealed, and statically cultured). It was found that after 48 hours, the transformants could consume 20 g/L glucose and carry out the accumulation of butanedioic acid. It was also discovered that compared with the other transformants, the butanedioic acid production of transformant #28 was obviously higher and the accumulation of by-products was significantly less. As shown in FIG. 2, the butanedioic acid production of transformant #28 can reach 0.77 g/L, the accumulation of by-product glycerol was 1.5 g/L, the accumulation of by-product lactic acid was 2.5 g/L, and the accumulation amounts of by-products acetic acid and citric acid were small. Butanedioic acid production of transformant #28 was 7.7 times that of the parent strain DLY-KMSP-0209M under the same fermentation conditions. Therefore, transformant #28 was selected for further study and named DLY-KMPMFF-0329.

*Kluyveromyces marxianus* DLY-KMPMFF-0329 was deposited in China General Microbiological Culture Collection Center (referred to as CGMCC, address: No. 3, Courtyard 1 Beichen West Road, Chaoyang District, Beijing, Institute of Microbiology, Chinese Academy of Sciences, Postal Code 100101) on April 3, 2023, and the classification name is *Kluyveromyces marxianus* with a deposit number of CGMCC No. 27009.

The experimental results above proved the feasibility of synthesizing butanedioic acid by using the *Kluyveromyces marxianus* via the reductive TCA pathway, and also indicated that the by-products were mainly composed of lactic acid, followed by glycerol, and then acetic acid. In view of that the dissolved oxygen level during the fermentation process may affect the accumulation of lactic acid, subsequent attempts were made to use micro-aerobic culture methods to inhibit lactic acid production and allow more pyruvic acid to flow to the reductive pathway of butanedioic acid. Therefore, subsequent studies were conducted on the effects of the reductive TCA pathway on butanedioic acid production under different oxygen levels, in order to perform the micro-aerobic fermentation within a range that enables the reductive TCA pathway to implement its catalytic function. Initially, the dissolved oxygen level was adjusted by changing the rotational speed of the shaking table during the shake flask exploration stage, and the dissolved oxygen control study was then conducted at the fermenter scale-up level.

### Example 4 Micro-aerobic fermentation of the strain for producing butanedioic acid via the reductive TCA pathway

Since the strain DLY-KMPMFF-0329 did not grow under the completely anaerobic conditions and lactic acid was generated. Therefore, the feasibility of producing butanedioic acid under micro-aerobic conditions was tested. 50 mL of YPD liquid medium was loaded into a 250 mL conical flask (the liquid level was marked), about 1.2 g of CaCO₃ (final concentration was about 24 g/L) was added and an oxygen limiting rubber plug was used. After continuous cultivation under the conditions of 37°C and rotational speed of 75 rpm for 7 days (the liquid level dropped slightly due to evaporation, and water was added to calibrate the volume), it was surprised to find by HPLC detection that the only residual metabolite was butanedioic acid, and there was almost no accumulation of by-products (FIG. 3). The production of the target product butanedioic acid was 3.89 g/L, and the yield was 0.195 g/g. The experimental results demonstrated that the effect of micro-aerobic conditions on the production of butanedioic acid and the accumulation of by-products was very obvious, so an experiment was performed to investigate an effect of different oxygen quantities on the butanedioic acid production.

### Example 5 Effect of different oxygen supply quantities on butanedioic acid production by the strain DLY-KMPMFF-0329

To explore the effect of oxygen supply rate (under oxygen-limited conditions) on butanedioic acid production, the fermentation experiments with different rotational speeds were designed and metabolites of the strains were detected. Under the conditions of a YPD culture medium (50 g/L of glucose) with the addition of CaCO₃ at a final concentration of 30 g/L, an oxygen limiting rubber plug was used, and the temperature of 37°C, the testing was performed by arranging the rotational speeds at 200, 150, 100, and 75 rpm, respectively, and the metabolites of each group of strains were detected by HPLC, the results were shown in FIG. 4. The production of butanedioic acid reached 7.5 g/L at XY-75r (oxygen-limited, 75 rpm). With the increase of rotational speed, the production rate of succinic acid was the fastest under the condition of XY-200r (oxygen-limited, 200 rpm), but the output was not as high as that of XY-75r. It indicated that the oxygen supply has a direct impact on the production of butanedioic acid, including an influence on the enzyme activity in the reductive TCA pathway (e.g., the enzyme activity of a key enzyme PYC2p that was sensitive to oxygen gas and catalyzes the production of oxaloacetic acid from pyruvic acid and HCO₃⁻), as well as the effect of oxygen gas on the glycolysis rate (the high oxygen supply will also affect growth of the strain).

As can be seen that in the above-mentioned micro-aerobic fermentation process, the consumption rate of glucose was relatively slow as a whole, and even under aerobic conditions, 96h was required to consume 50g/L of glucose, the reason may be that the metabolic flow biased towards butanedioic acid production after the addition of CaCO₃, resulting in a lower environmental pH. In addition, when the rotational speed was gradually reduced, the glucose consumption characteristics approached the straight line from the curve, and the consumption of glucose every 24h was almost identical. This may be caused by that the speed of pyruvic acid produced by the metabolism of glucose under a specific oxygen gas supply was equivalent with the flow rate of pyruvic acid to the synthesis of succinic acid. As illustrated by FIG. 4, there was a certain margin between the glucose consumption curves of the rotational speeds of 150rpm and 100rpm, thus it was inferred that the rotational speeds of 125rpm may be more conducive to balancing the glucose consumption rate and the butanedioic acid yield, and reducing the generation of by-products. In regard to the generation of by-products (e.g., lactic acid, glycerol), it was speculated that the generation of by-products mainly attributed to the overflow of pyruvic acid (the production rate of pyruvic acid was greater than the consumption rate of pyruvic acid for the butanedioic acid production). As shown in c) of FIG. 4, the production of lactic acid gradually decreased along with an increase of rotational speed under the oxygen-limited conditions, and the overall maximum production of lactic acid was about 3g/L, and the lactic acid will be consumed as a carbon source in the later stage. Under the conditions with more oxygen gas, the pyruvic acid metabolism was more inclined to the respiratory chain with higher production capacity. The above results once again proved the importance of oxygen gas supply control for the succinic acid production. As shown in d) of FIG. 4, the production of glycerol decreases with an increase of rotational speed under the oxygen-limited conditions, and the maximum production of glycerol did not exceed 2g/L. The production of glycerol required the consumption of one molecule of NADH, thus the glycerol competed with butanedioic acid in terms of NADH and carbon sources. Theoretically, the NADH consumed by each additional 1g of glycerol as a by-product can convert 1.46g of malic acid into 1.28g of butanedioic acid, which not only reduced the by-product malic acid, but also increased the production and yield of butanedioic acid. Based on the comprehensive results of FIG. 4, the butanedioic acid production was high at the rational speed of 200rpm under the oxygen-limited conditions, and the accumulation of each by-product was low. In addition, the optimization results of CaCO₃ addition amount showed that an addition of 15g/L CaCO₃ had a better effect on increasing the butanedioic acid production.

### Example 6 Production and detection of butanedioic acid by strain DLY-KMPMFF-0329 in shake flask fermentation

In order to shorten the culture period, the shake flask fermentation of strain DLY-KMPMFF-0329 for the production of butanedioic acid was carried out under the conditions that the culture medium was YPD₅₀ (glucose 50g/L) + 15g/L CaCO₃, 250mL triangular bottle filled to 50mL, the rotational speed was 200 rpm and the temperature was 37°C. The results showed that the production of SA reached 9.73g/L and the yield was 0.24g/g after the shake flask fermentation for 96h. Under the conditions that the culture medium was YPD₈₀ (glucose 80g/L) + 15g/L CaCO₃, after feeding 15g/L of CaCO₃ in 72h, the glucose was completely consumed at 168h, and SA production reached 16.5g/L, the SA yield was 0.23g/g. However, when the initial glucose concentration was 100g/L, the production and yield of SA were not the highest, as shown in FIG. 5 a). Surprisingly, under the conditions that the culture medium was YD₅₀ (0g/L peptone + 10g/L yeast powder + 50g/L glucose) + 15g/L CaCO₃, the rotation speed was 200rpm, and the temperature was 37°C, the SA production reached 13.2g/L at 120h, and the yield was 0.26g/g, as shown in FIG. 5 b). It demonstrated that an increased carbon-nitrogen ratio was beneficial to the production of SA, and also provided a desirable reference for reducing the culture medium cost for producing SA in the future.

### Example 7 Production and detection of butanedioic acid by strain DLY-KMPMFF-0329 in fermenter fermentation

It was discovered through the previous shake flask experiments that the dissolved oxygen level has a very important influence on the production and yield of SA by the strain DLY-KMPMFF-0329. In order to explore the conditions for the fermentation and production of SA by the strain DLY-KMPMFF-0329 in a fermenter, the fermentation and production of SA were carried out in a 5L fermenter (FIG. 6). The fermentation medium formula was as follows: peptone 20g/L, yeast powder 10g/L, glucose 50g/L, calcium carbonate 15g/L, and the feeding strategy was to add mother liquor glucose (500g/L) once each time the glucose was exhausted. The temperature was 37°C, and the DO level was controlled by manually adjusting the rotational speed and ventilation volume (generally not more than 1%). Continuous sampling was performed and HPLC was used to detect changes in metabolites as the basis for adjusting the rotational speed and ventilation volume. As of 250h of cultivation, a total of 175 g/L of glucose was consumed. Four material replenishments were conducted during the process, as shown in FIG. 6 a). 37.5 g/L CaCO₃ was added in three batches, wherein the first batch was 15 g/L CaCO₃ added into the medium before fermentation, the second batch was 15 g/L CaCO₃ added at 96 h of cultivation, and the third batch was 7.5 g/L CaCO₃ added at 144 h of cultivation (the need to add CaCO₃ can be determined based on the microscopic examination and pH changes in the fermentation liquid). The production of butanedioic acid was 25.2 g/L, and the yield of butanedioic acid was 0.144 g/g glucose.

In addition, the fermentation cycle was relatively long and the accumulation of SA began to accelerate in the second half of the fermentation process. It was speculated that the main reasons resided in an increased number of thallus and the improvement of dissolved oxygen, which gradually increased from 100 mL/min (the dissolved oxygen DO value was about 0%) to 250 mL/min and further hiked to 1 vvm (DO was about 1%). The slow accumulation rate of SA in the early stage may be due to the slow growth of biomass caused by insufficient oxygen gas. After the dissolved oxygen was improved, the glucose consumption was significantly accelerated, the SA accumulation rate was significantly improved, and the accumulation amount of lactic acid began to decrease till zero, as shown in FIG. 6 c). The above results suggested that the growth stage can be completed quickly before entering the SA production stage.

As illustrated by the results of above examples, the *Kluyveromyces marxianus* DLY-KMPMFF-0329 provided by the invention not only has good tolerance to low pH and high concentration of butanedioic acid, but also can efficiently produce butanedioic acid via the reductive TCA pathway; the strain produces butanedioic acid by fermentation through cytoplasmic expression, which greatly improves the production and yield of butanedioic acid. In addition, by combination with the preferred fermentation modes and conditions of the present invention (such as controlling the dissolved oxygen during the fermentation process, i.e., carrying out the fermentation under the oxygen-limited conditions), the growth rate of DLY-KMPMFF-0329 is increased, such that it can quickly enter the SA production stage, shorten the SA fermentive production cycle, and improve production efficiency.

Finally, it should be noted that the above examples are only used to illustrate the technical solutions of the present invention, instead of imposing limitations thereto. Although the present invention has been described in detail with reference to the aforementioned examples, those skilled in the art should understand that they still can make amendments on the technical solutions described in the aforementioned examples, or carry out the equivalent replacements with respect to a portion of the technical features therein. However, these modifications or replacements do not deviate the essence of the corresponding technical solutions from the scope of the technical solutions of the examples in the present invention.

### Sequence listing

**SEQ ID NO.1** 
MSTQNDLAGLRDNSNLLGEKNKILVANRGEIPIRIFRTAHELSMKTVAIYSHEDRLSMHRL
KADEAYVIGEPGKYTPVGAYLAIDEIIKIAQLHGVSFIHPGYGFLSENSEFAKKVADSGITW
VGPPADVIDAVGDKVSARNLAERADVPVVPGTPGPIETVEEAVEFVEKYGYPVIIKAAFGG
GGRGMRVVREGDDIADAFQRAKSEAVTAFGNGTCFVERFLDKPKHIEVQLLADHYGNVI
HLFERDCSVQRRHQKVVEVAPAKTLPESVRNAILTDAVKLAKEAGYRNAGTAEFLVDNQN
RHYFIEINPRIQVEHTITEEITGIDIVAAQIQIAAGASLEQLGLLQDRITTRGFAIQCRITTEDPS
KNFQPDTGRIDVYRSAGGNGVRLDGGNAFAGSVISPHYDSMLVKCSCSGSTYEIVRRKML
RALIEFRIRGVKTNIPFLLTLLTHPVFKSGDYWTTFIDDTPQLFEMVSSQNRAQKLLHYLAD
LAVNGSSIKGQIGLPKLKTHPTIPHLHKADGSILDVSAKPPAGWRDVLLQHGPEEFAKQVR
KFKGTLLMDTTWRDAHQSLLATRVRTYDLAAIAPTTAHALSGAFALECWGGATFDVSMR
FLHEDPWERLRTLRKLVPNIPFQMLLRGANGVAYSSLPDNAIDHFVKQAKDNGVDIFRVF
DALNDLEQLTVGVDAVKKAGGVVEATICYSGDMLAPGKKYNLDYYLDIVEQVVKRGTHI
LGIKDMAGTLKPSAAKLLIGSIRTKYPDLPIHVHTHDSAGTGVASMAACAFAGADVVDVA
TNSMSGMTSQPSVNALLAALDGEIDCNVNVSYISQLDAYWAEMRLLYSCFEADLKGPDPE
VYVHEIPGGQLTNLLFQAQQLGLGEQWAETKRAYREANLLLGDVVKVTPTSKVVGDLAQ
FMVTNKLTSDDVKRLASSLDFPDSVMDFFEGLIGQPYGGFPEPLRSDVLKNKRRKLTKRP
GLELAPFDLEGIKEDLTNRFGDIDDCDVASYNMYPKVYEDFRKIREKYGDLSVLPTKNFLS
PPSIGEEIVVTIEQGKTLIIKPQAIGDLNKETGIREVYFELNGELRKVSVADRSQKVETISKPK
ADAHDPFQVGSPMAGVVVEVKVHKGSLISKGQPVAVLSAMKMEMVISSPSDGQVKEVLV
KDGENVDASDLLVVLEEAPAKE
**SEQ ID NO.2** 
MVSVAVLGSSGGIGQPLSLLLKLDPRVSSLRLYDLKMSHGIATDLSHMDSNSICEGFNTDEI
ALALKGAQIVVIPAGVPRKPGMSRDDLFKINAKIIKSLALQIAEHAPEARVLVISNPVNSLV
PIVYETLKSVGKFEPGKVMGITTLDIIRSHTFLVDVLGRKAYSVEKLRSAVTVVGGHSGETI
VPIFTDQKFYRRLRDRELYDAYVHRVQFGGDEVVKAKDGSGSATLSMAWAGYSFVKQLL
NSLHLETGEDVHPIPTFVYLPGLPGGKELQQKLGTSVEFFAAPVKLSKGIVVEVEHDWVD
KLNDAEKKLIAKCLPILDKNIKKGLAFSQQ
**SEQ ID NO.3** 
MSVRMVSYRVETDAFGEIKVPADKYWGAQTQRSFQNFKIGGAREKMPEPIVKAFGVLKK
SAAIVNENLGTLDPAVAEPIKKAADEVAQGKLMDHFPLVVFQTGSGTQSNMNANEVISNR
AIELIGGELGSKQIHPNNHCNQAQSSNDTFPSVMHIAAVTEITRHLLPELVALKDALKKKSD
EFQHIVKIGRTHLQDATPLTLGQEFSGYVQQVENGIIRVEQSLHHLKFLAQGGTAVGTGLN
TKVGFAEKIAEQVSKETGIDFKTAPNKFEALAAHDAIVEASGALNTLAVSLFKIANDIRYLG
SGPRCGYGELSLPENEPGSSIMPGKVNPTQNEALTQVCVQVMGNHAAITFAGASGQFELN
VFKPVMISNLLSSIRLLGDASNSFRIHCVEGIQANEDRISKLLHESLMLVTALNPKIGYDAA
SKVAKNAHKKGITLKESALELGVLSSEEFDEWVIPEKMIGPKE
**SEQ ID NO.4** 
MVDGRSSASIVAVDPERAARERDAAARALLQDSPLHTTMQYATSGLELTVPYALKVVASA
DTFDRAKEVADEVLRCAWQLADTVLNSFNPNSEVSLVGRLPVGQKHQMSAPLKRVMAC
CQRVYNSSAGCFDPSTAPVAKALREIALGKERNNACLEALTQACTLPNSFVIDFEAGTISRK
HEHASLDLGGVSKGYIVDYVIDNINAAGFQNVFFDWGGDCRASGMNARNTPWVVGITRP
PSLDMLRNPPKEASYISVISLDNEALATSGDYENLIYTADDKPLTCTYDWKAKELMKPSQS
NIAQVSVKCYSAMYADALATACFIKRDPAKVRQLLDGWRYVRDTVRDYRVYVRENERVA
KMFEIATEDAEMRKRRISNTLPARVIVVGGGLAGLSAAIEAAGCGAQVVLMEKEAKLGG
NSAKATSGINGWGTRAQAKASIVDGGKYFERDTYKSGIGGNTDPALVKTLSMKSADAIG
WLTSLGVPLTVLSQLGGHSRKRTHRAPDKKDGTPLPIGFTIMKTLEDHVRGNLSGRITIME
NCSVTSLLSETKERPDGTKQIRVTGVEFTQAGSGKTTILADAVILATGGFSNDKTADSLLRE
HAPHLVNFPTTNGPWATGDGVKLAQRLGAQLVDMDKVQLHPTGLINPKDPANPTKFLGP
EALRGSGGVLLNKQGKRFVNELDLRSVVSKAIMEQGAEYPGSGGSMFAYCVLNAAAQK
 
LFGVSSHEFYWKKMGLFVKADTMRDLAALIGCPVESVQQTLEEYERLSTSQRSCPITRKS
VYPCVLGTKGPYYVAFVTPSIHYTMGGCLISPSAEIQMKNTSSRAPLSHSNPILGLFGAGEV
TGGVHGGNRLGGNSLLECVVFGRIAGDRASTILQRKSSALSFKVWTTVVLREVREGGVY
GAGSRVLRFNLPGALQRSGLSLGQFIAIRGDWDGQQLIGYYSPITLPDDLGMIDILARSDK
GTLREWISALEPGDAVEMKACGGLVIERRLSDKHFVFMGHIINKLCLIAGGTGVAPMLQII
KAAFMKPFIDTLESVHLIYAAEDVTELTYREVLEERRRESRGKFKKTFVLNRPPPLWTDGV
GFIDRGILTNHVQPPSDNLLVAICGPPVMQRIVKATLKTLGYNMNLVRTVDETEPSGS
**SEQ ID NO.5**

ATGGTCGATGGTAGATCTTCTGCTTCTATCGTTGCTGTTGACCCAGAAAGAGCTGCTAG
AGAAAGAGACGCTGCTGCTAGAGCTTTGTTGCAAGACTCCCCATTGCACACCACCATG
CAATACGCTACTTCCGGTTTGGAATTGACCGTTCCATACGCTTTGAAGGTTGTTGCTTC
CGCTGACACCTTCGACAGAGCTAAGGAAGTTGCTGACGAAGTTTTGAGATGTGCTTGG
CAATTGGCTGACACTGTTTTGAACTCTTTCAACCCAAACTCTGAAGTTTCTTTGGTTGG
TAGATTGCCAGTTGGTCAAAAGCACCAAATGTCTGCTCCATTGAAGAGAGTTATGGCTT
GTTGTCAAAGAGTTTACAACTCTTCTGCTGGTTGTTTCGACCCATCTACTGCTCCAGTT
GCTAAGGCTTTGAGAGAAATCGCTTTGGGTAAGGAAAGAAACAACGCTTGTTTGGAA
GCTCTAACTCAAGCCTGTACCTTGCCAAACTCTTTCGTTATTGACTTCGAAGCTGGTAC
TATCTCTAGAAAGCACGAACACGCTTCTTTGGACTTGGGTGGTGTCTCTAAGGGTTACA
TTGTCGATTACGTTATTGACAACATTAACGCTGCTGGTTTCCAAAACGTTTTCTTCGACT
GGGGTGGTGACTGTAGAGCTTCTGGTATGAACGCTAGAAACACCCCATGGGTTGTTGG
TATTACCAGACCACCATCTTTGGACATGTTGAGAAACCCACCAAAGGAAGCTAGTTACA
TCTCTGTTATTTCTTTGGATAACGAAGCTCTAGCTACCTCTGGTGACTACGAAAACTTGA
TCTACACCGCTGACGACAAGCCATTGACCTGTACCTACGACTGGAAGGCTAAGGAATT
GATGAAGCCATCCCAATCTAACATCGCTCAAGTTTCTGTTAAGTGTTACTCTGCTATGTA
CGCTGACGCTTTGGCTACCGCTTGTTTCATCAAGAGAGATCCAGCTAAGGTTAGACAAT
TGTTGGACGGTTGGAGATACGTTAGAGACACCGTTAGAGACTACAGAGTTTACGTTAG
AGAAAACGAAAGAGTTGCTAAGATGTTCGAAATCGCTACCGAAGACGCTGAAATGAG
AAAGAGAAGAATCTCTAACACCTTGCCAGCTAGAGTTATCGTTGTTGGTGGTGGTTTG
GCTGGTTTGTCTGCTGCTATTGAAGCTGCTGGTTGTGGTGCTCAAGTTGTTTTGATGGA
AAAGGAAGCTAAGTTGGGTGGTAACTCTGCTAAGGCTACCTCTGGTATTAACGGTTGG
GGTACTAGAGCTCAAGCTAAGGCTTCTATTGTTGACGGTGGTAAGTACTTCGAAAGAG
ACACCTACAAGTCCGGTATTGGTGGTAACACTGACCCAGCTTTGGTTAAGACCTTGTCT
ATGAAGTCCGCTGACGCTATCGGTTGGTTGACCTCTTTGGGTGTCCCATTGACCGTTTT
GTCCCAATTGGGTGGTCACTCTAGAAAGAGAACCCATAGAGCTCCAGACAAGAAGGA
CGGTACTCCATTGCCAATTGGTTTCACTATCATGAAGACTTTGGAAGACCACGTCAGAG
GTAACTTGTCTGGTAGAATCACCATCATGGAAAACTGTTCCGTTACCTCTTTGTTGTCT
GAAACCAAGGAAAGACCAGACGGTACTAAGCAAATCAGAGTTACCGGTGTTGAATTTA
CTCAAGCTGGTTCTGGTAAGACCACCATCTTGGCTGACGCTGTTATCTTGGCTACCGGT
GGTTTCTCTAACGACAAGACTGCTGACTCTTTGTTGAGAGAACACGCTCCACACTTGG
TTAACTTCCCAACCACTAACGGTCCATGGGCTACTGGTGATGGTGTTAAGTTGGCTCAA
AGATTGGGTGCTCAATTGGTTGACATGGACAAGGTTCAATTGCACCCAACCGGTTTGAT
CAACCCAAAGGACCCAGCTAACCCAACCAAGTTCTTGGGTCCAGAAGCTCTAAGAGG
TTCCGGTGGTGTTTTGTTGAACAAGCAAGGTAAGAGATTCGTTAACGAATTGGATTTGA
GATCCGTTGTTTCTAAGGCTATCATGGAACAAGGTGCTGAATACCCAGGTTCTGGTGGT
TCTATGTTCGCTTACTGTGTCTTGAACGCTGCTGCTCAAAAGTTGTTCGGTGTTTCTTCT
CACGAATTTTACTGGAAGAAGATGGGTTTGTTCGTTAAGGCTGATACTATGAGAGATTT
GGCTGCTTTGATCGGTTGTCCAGTTGAATCTGTTCAACAAACTTTGGAAGAATACGAA
AGATTGTCTACCTCTCAAAGATCTTGTCCAATTACCAGAAAGTCTGTTTACCCATGTGTT
TTGGGTACTAAGGGTCCATACTACGTTGCTTTCGTTACCCCATCTATTCACTACACCATG
GGTGGTTGTTTGATCTCTCCATCTGCTGAAATCCAAATGAAGAACACCTCTTCTAGAGC
TCCATTGTCTCACTCTAACCCAATTTTGGGTTTGTTCGGTGCTGGTGAAGTTACCGGTG
GTGTTCACGGTGGTAACAGATTGGGTGGTAACTCCTTGTTGGAATGTGTTGTTTTCGGT
AGAATCGCTGGTGACAGAGCTTCTACTATTTTGCAAAGAAAGTCTTCTGCTTTGTCTTT
CAAGGTTTGGACTACTGTTGTTTTGAGAGAAGTTAGAGAAGGTGGTGTTTACGGTGCT
GGTTCTAGAGTTTTGAGATTCAACTTGCCAGGTGCTTTGCAAAGATCTGGTTTGTCTTT

GGGTCAATTCATCGCTATTAGAGGTGACTGGGATGGTCAACAATTGATCGGTTACTACT
CTCCAATCACCTTGCCAGACGACTTGGGTATGATCGACATTTTGGCTAGATCTGATAAG
GGTACTTTGAGAGAATGGATTTCTGCTTTGGAACCAGGTGACGCTGTTGAAATGAAGG
CTTGTGGTGGTTTGGTTATTGAAAGAAGATTGTCCGACAAGCATTTCGTTTTCATGGGT
CATATCATCAACAAGTTGTGTTTGATCGCTGGTGGTACTGGTGTTGCTCCAATGTTGCA
AATTATTAAGGCTGCTTTCATGAAGCCATTCATCGACACCTTGGAATCTGTTCACTTGAT
CTACGCTGCTGAAGACGTTACCGAATTGACTTACAGAGAAGTTTTGGAAGAAAGAAG
AAGAGAATCTAGAGGTAAGTTCAAGAAGACCTTCGTTTTGAACAGACCACCACCATTG
TGGACCGATGGTGTTGGTTTCATCGACAGAGGTATCTTGACCAACCACGTTCAACCAC
CATCCGACAACTTGTTGGTTGCTATCTGTGGTCCACCAGTTATGCAAAGAATCGTTAAG
GCTACCTTGAAGACCTTGGGTTACAACATGAACTTGGTTAGAACCGTTGACGAAACCG
AACCATCTGGTTCCTAA

## Claims

1. *Kluyveromyces marxianus* DLY-KMPMFF-0329 **characterized in that** the *Kluyveromyces marxianus* DLY-KMPMFF-0329 is deposited in China General Microbiological Culture Collection Center with a deposit number of CGMCC No.27009.

2. *Kluyveromyces marxianus* **characterized in that** the *Kluyveromyces marxianus* comprises a gene expressing a fumaratec reductase FRDg derived from *Trypanosoma brucei*.

3. The *Kluyveromyces marxianus* according to claim 2, wherein amino acid sequence of the fumaratec reductase FRDg has identity not less than 70%, preferably not less than 80%, more preferably not less than 90% with the amino acid sequence as set forth in SEQ ID No. 4;
preferably, the amino acid sequence of fumaraic reductase FRDg has identity of 95-100% with an amino acid sequence as set forth in SEQ ID No. 4.

4. The *Kluyveromyces marxianus* according to claim 2 or 3, wherein genome of the *Kluyveromyces marxianus* includes an encoding gene of fumaratec reductase FRDg with a nucleotide sequence having identity more than 60% with the nucleotide sequence as set forth in SEQ ID No. 5.

5. The *Kluyveromyces marxianus* according to claim 4, wherein encoding gene of the fumaratec reductase FRDg has identity not less than 70%, more preferably not less than 80%, further preferably not less than 90% with the nucleotide sequence as set forth in SEQ ID No. 5;
preferably, encoding gene of the fumaraic reductase FRDg has identity of 95-100% with the nucleotide sequence as set forth in SEQ ID No. 5.

6. The *Kluyveromyces marxianus* bacterium according to any one of claims 2-5, wherein the *Kluyveromyces marxianus* differs from *Kluyveromyces marxianus* with a deposit number of CGMCC No.24398 in the inactivated encoding gene of malic enzyme MAE1;
and/or, the *Kluyveromyces marxianus* bacterium differs from *Kluyveromyces marxianus* with a deposit number of CGMCC No.24398 in the inactivated encoding gene of pyruvate decarboxylase PDC1;
preferably, the *Kluyveromyces marxianus* is DLY-KMPMFF-0329 with a deposit number of CGMCC No 27009.

7. A microorganism preparation **characterized in that** the microorganism preparation comprises *Kluyveromyces marxianus* according to any one of claims 1-6 and auxiliary ingredients.

8. The microorganism preparation according to claim 7, wherein the auxiliary ingredients comprise at least one selected from the group consisting of a protective agent, a buffering agent and a stabilizer.

9. A use of the *Kluyveromyces marxianus* according to any one of claims 1-6 or the microorganism preparations according to claims 7 or 8 in anyone as following:
(1) use in fermentive production of an organic acid or derivatives thereof;
(2) use in breeding and/or constructing production strain of an organic acid or derivatives thereof.

10. The use according to claim 9, wherein the organic acid is butanedioic acid.

11. A method for fermentive production of butanedioic acid **characterized in that** the method comprises a step of culturing the *Kluyveromyces marxianus* according to any one of claims 1-6.

12. The method according to claim 11, wherein the culturing comprises a fermentation stage comprising a step of culturing the *Kluyveromyces marxianus* under oxygen-limited conditions;
preferably, the oxygen-limited conditions serve to control dissolved oxygen DO to below 2%.

13. The method according to claim 11 or 12, wherein during the fermentation cultivation stage, carbonate is added at the fermentation initiation stage and/or during fermentation process;
preferably, the carbonate is added in an amount of 10-50g/L, preferably the carbonate comprises at least one selected from the group consisting of calcium carbonate, sodium carbonate, potassium carbonate and ammonium carbonate.

14. The method according to any one of claims 11-13, wherein the *Kluyveromyces marxianus* is *Kluyveromyces marxianus* DLY-KMPMFF-0329 with a deposit number of CGMCC No. 27009.
